# FASCICULE DE BREVET EUROPEEN

(11) **EP 0 595 690 B1**
(45) Date de publication et mention de la délivrance du brevet: **08.01.1997**
(21) Numéro de dépôt: 93402589.1
(22) Date de dépôt: 21.10.1993
(51) Int. Cl.: A61K 7/48, A61K 7/00, A45D 40/00

(54) **Produit cosmétique à potentiel redox stabilisé**
Kosmetisches Produkt mit stabilisiertem Redoxpotential
Cosmetic product with stabilized redox potential

(30) Priorité: 26.10.1992 FR 9212745
(43) Date de publication de la demande: 04.05.1994
(73) Titulaire: DANIEL JOUVANCE, F-13763 Aix Les Milles Cédex (FR)
(72) Inventeur: Rocher, Daniel, F-13290 Les Milles (FR); Noel, Hugues, F-13250 St Chamas (FR)
(74) Mandataire: Polus, Camille

(56) Documents cités:
- EP-A- 0 326 143
- FR-A- 1 247 588
- FR-A- 1 352 217
- PATENT ABSTRACTS OF JAPAN vol. 12, no. 22 (C-470)(2869) & JP-A-62 175 419 (TOMOJI TANAKA)

## Description

La présente invention concerne un produit cosmétique à potentiel redox stabilisé comprenant un sel métallique et le métal correspondant.

La seule incorporation de sels métalliques dans des compositions cosmétiques est connue et utilisée depuis longtemps (voir par exemple FR-A-1247588). La seule addition de métaux à des fins décoratives ou pour une action physiologique (écran, réflecteur) a également été décrite. Cependant la demanderesse n'a trouvé aucune description d'une incorporation simultanée de sel métallique et de son métal correspondant dans l'état de la technique des produits cosmétiques.

L'objet de la présente invention est de fournir un produit cosmétique à potentiel rédox stabilisé, grâce à une mise en contact l'un avec l'autre d'une composition cosmétique, comprenant en milieu aqueux un sel métallique hydrosoluble, et le métal correspondant au sel métallique hydrosoluble.

La composition cosmétique peut être toute composition cosmétique classique contenant un sel métallique et comprenant une base cosmétique aqueuse ou hydroalcoolique.

Dans la présente invention par sel métallique, on désigne aussi bien un sel simple d'un métal qu'un complexe métallique. Comme exemple de complexes métalliques on peut citer ceux formés avec l'ammoniaque, EDTA, HEDTA, DTPA, l'acide orotique, l'acide urique, l'acide phytique, l'acide étidronique et/ou leurs sels.

Le métal du sel métallique peut être notamment le zinc, le cuivre, le fer, le molybdène, le vanadium, le sélénium, le nickel, le manganèse et le titane.

Le constituant métallique associé à la composition cosmétique, selon la présente invention, peut être sous forme de poudre et/ou de grenaille, dispersée(s) dans la composition cosmétique. Il peut être aussi sous forme de fil, par exemple un fil fixé au dispositif d'obturation du contenant du produit cosmétique de la présente invention, ou sous forme d'une pièce métallique permettant notamment l'application de la composition cosmétique de la présente invention sur la peau et/ou les phanères, cette pièce pouvant être moulée, extrudée ou frittée, fixée ou non à une partie du contenant du produit cosmétique. Le contenant du produit cosmétique peut d'ailleurs également être constitué du métal ou d'un alliage du métal dont le sel est incorporé à la composition cosmétique.

La stabilité du potentiel rédox du produit cosmétique de la présente invention permet par exemple une conservation remarquable des propriétés de la composition cosmétique, notamment des propriétés physiologiques.

Les métaux préférés de la présente invention sont généralement ceux dont les formes ionisées complexées ou non présentent une activité physiologique (par exemple en association dans des métallo enzymes, des métallo protéines, des hemes) c'est à dire notamment le zinc, le cuivre, le fer, le molybdène, le vanadium, le sélénium, le nickel et le manganèse. Mais du point de vue de la protection contre l'oxydation on préfère des métaux tels que le zinc, le vanadium et le titane. La proportion préférée de sel métallique, exprimée en métal dans la composition, est comprise entre 10 ppm et 1%.

On trouvera ci-dessous deux exemples de produit cosmétique selon la présente invention.

| **CREME DE SOIN ANTIRIDES** | |
|---|---|
| Eau déminéralisée | qsp 100 |
| Pyrolidonecarboxylate de zinc | 1,00 |
| Glycérine | 1,00 |
| Butanediol-1,3 | 5,00 |
| Lactate de sodium | 0,50 |
| Cetylsulfate de sodium | 2,50 |
| Conservateur | qsp |
| Paramethoxycinnamate d'isoamyle | 2,00 |
| Gamaorizanol | 1,00 |
| Laurate d'ethylhexyle | 17,00 |
| Stearate de pentaerythritol | 3,00 |
| Diethylhexanoate de propanediol-1,2 | 6,50 |
| Huile de vaseline | 8,00 |
| Huile de jojoba | 2,00 |
| Stearate de PEG 20 | 0,75 |
| Monolinoleate de glycérol | 1,30 |
| Monostearate de sorbitan | 1,50 |
| Dodecamethylcyclohexasiloxane | 4,00 |
| DL x tocophérol | 0,10 |
| Palmitate de rétinol | 0,15 |
| Huile de jojoba | 2,40 |
| Polyacrylamide | 0,60 |
| Acide désoxyribonucléique | 0,30 |
| Collagène (solution 0,3 %) | 3,00 |
| Parfum | qs |
| Associée à un fil de zinc pur fixé au couvercle du pot. | |

| **CREME SOLAIRE** | |
|---|---|
| Eau distillée | qsq |
| Propanediol-1,2 | 2,50 |
| Acide stéarique éthoxylé 100 OE | 2,00 |
| Acide stéarique éthoxylé 30 OE | 2,20 |
| Acide orthophosphorique | 0,09 |
| Sulfate de magnésium | 0,50 |
| Pyrolidonecarboxylate de cuivre | 0,20 |
| Hydroxyde de sodium | 0,74 |
| Acide phénylbenzimidazolesulfonique | 3,00 |
| Décamethylcyclopentasiloxane | 4,00 |
| Huile de coprah hydrogénée | 8,00 |
| Huile de sésame | 6,00 |
| Monostéarate de pentaerythritol | 5,00 |
| Paramethoxycinnamate d'éthylhexyle | 4,00 |
| Monostéarate de glycérol | 4,00 |
| Beurre de karité | 3,00 |
| Terbutylmethoxydibenzoylmethane | 3,00 |
| Cire de carnauba | 2,00 |
| Oléate de décyle | 2,00 |
| Conservateur | 1,20 |
| Eau de mer | 4,00 |
| Extrait de diginéa | 1,00 |
| Parfum | 0,30 |
| Associée à un fil de cuivre pur. | |

Il est clair que ces exemples ne sont pas limitatifs quant aux autres possibilités d'association, selon la présente invention, d'un sel métallique avec son métal correspondant.

## Revendications

1. Produit cosmétique à potentiel rédox stabilisé caractérisé en ce qu'il comprend, en contact l'un avec l'autre, une composition cosmétique comprenant, en milieu aqueux, un sel métallique hydrosoluble et le métal correspondant à ce sel métallique hydrosoluble .

2. Produit cosmétique selon la revendication 1, caractérisé en ce que le sel hydrosoluble est un complexe métallique.

3. Produit cosmétique selon les revendications 1 et 2, caractérisé en ce que le complexe métallique est un complexe formé avec l'ammoniaque, EDTA, HEDTA, DTPA, l'acide orotique, l'acide urique, l'acide phytique, l'acide étidronique et/ou leurs sels.

4. Produit cosmétique selon la revendication 1, caractérisé en ce que la proportion du sel hydrosoluble, exprimée en métal, est comprise entre 10 ppm et 1%.

5. Produit cosmétique selon la revendication 1, caractérisé en ce que le métal, correspondant au sel métallique hydrosoluble, est choisi parmi le zinc, le cuivre, le fer, le molybdène, le vanadium, le sélénium, le nickel, le manganèse et le titane.

6. Produit cosmétique selon la revendication 1, caractérisé en ce que le métal est sous forme de poudre dispersée dans la composition cosmétique.

7. Produit cosmétique selon la revendication 1, caractérisé en ce que le métal est sous forme de grenaille dispersée dans la composition cosmétique.

8. Produit cosmétique selon la revendication 1, caractérisé en ce que le métal est un fil fixé au dispositif d'obturation du contenant du produit cosmétique.

9. Produit cosmétique selon la revendication 1, caractérisé en ce que le métal constitue une partie du contenant du produit cosmétique.

10. Produit cosmétique selon la revendication 1, caractérisé en ce que le métal est sous la forme d'une pièce pour une application de la composition sur la peau et/ou sur les phanères.

## Claims

1. Cosmetic product having a stabilised redox potential, characterised in that it comprises, in contact with one another, a cosmetic composition comprising, in aqueous medium, a water-soluble metal salt and the metal corresponding to this water-soluble metal salt.

2. Cosmetic product according to claim 1, characterised in that the water-soluble salt is a metal complex.

3. Cosmetic product according to claims 1 and 2, characterised in that the metal complex is a complex formed with ammonia, EDTA, HEDTA, DTPA, orotic acid, uric acid, phytic acid, etidronic acid and/or the salts thereof.

4. Cosmetic product according to claim 1, characterised in that the proportion of the water-soluble salt, expressed as a metal, is between 10 ppm and 1%.

5. Cosmetic product according to claim 1, characterised in that the metal corresponding to the water-soluble metal salt is selected from zinc, copper, iron, molybdenum, vanadium, selenium, nickel, manganese and titanium.

6. Cosmetic product according to claim 1, characterised in that the metal is in the form of a powder dispersed in the cosmetic composition.

7. Cosmetic product according to claim 1, characterised in that the metal is in the form of filings dispersed in the cosmetic composition.

8. Cosmetic product according to claim 1, characterised in that the metal is a wire fixed to the closure device for the container for the cosmetic product.

9. Cosmetic product according to claim 1, characterised in that the metal constitutes part of the container for the cosmetic product.

10. Cosmetic product according to claim 1, characterised in that the metal is in the form of a component for applying the composition to the skin and/or superficial body parts.

## Patentansprüche

1. Kosmetisches Produkt mit stabilisiertem Redox-Potential, dadurch gekennzeichnet, daß es umfaßt eine kosmetische Zusammensetzung, die in einem wäßrigen Medium enthält ein wasserlösliches Metallsalz, und das diesem wasserlöslichen Metallsalz entsprechende Metall, die miteinander in Kontakt stehen.

2. Kosmetisches Produkt nach Anspruch 1, dadurch gekennzeichnet, daß das wasserlösliche Salz ein Metallkomplex ist.

3. Kosmetisches Produkt nach den Ansprüchen 1 und 2, dadurch gekennzeichnet, daß der Metallkomplex ein Komplex ist, der gebildet worden ist mit Ammoniak, EDTA, HEDTA, DTPA, Orotsäure, Harnsäure, Phytinsäure, Etidronsäure und/oder ihren Salzen.

4. Kosmetisches Produkt nach Anspruch 1, dadurch gekennzeichnet, daß der Mengenanteil des wasserlöslichen Salzes, ausgedrückt als Metall, zwischen 10 ppm und 1 % liegt.

5. Kosmetisches Produkt nach Anspruch 1, dadurch gekennzeichnet, daß das Metall, das dem wasserlöslichen Metallsalz entspricht, ausgewählt wird aus der Gruppe Zink, Kupfer, Eisen, Molybdän, Vanadin, Selen, Nickel, Mangan und Titan.

6. Kosmetisches Produkt nach Anspruch 1, dadurch gekennzeichnet, daß das Metall in Form eines Pulvers vorliegt, das in der kosmetischen Zusammensetzung dispergiert ist.

7. Kosmetisches Produkt nach Anspruch 1, dadurch gekennzeichnet, daß das Metall in Form von Granalien vorliegt, die in der kosmetischen Zusammensetzung dispergiert sind.

8. Kosmetisches Produkt nach Anspruch 1, dadurch gekennzeichnet, daß das Metall ein Draht (Faden) ist, der an der Verschlußeinrichtung des Behälters für das kosmetische Produkt befestigt ist.

9. Kosmetisches Produkt nach Anspruch 1, dadurch gekennzeichnet, daß das Metall einen Teil des Behälters für das kosmetische Produkt darstellt.

10. Kosmetisches Produkt nach Anspruch 1, dadurch gekennzeichnet, daß das Metall in Form eines Stückes für den Auftrag der Zusammensetzung auf die Haut und/oder auf die Phaneren vorliegt.
